# FASCICULE DE BREVET EUROPEEN

(11) **EP 4 016 040 B1**
(45) Date de publication et mention de la délivrance du brevet: **25.09.2024**
(21) Numéro de dépôt: 21215170.8
(22) Date de dépôt: 16.12.2021
(51) Int. Cl.: G01N 1/12, G01N 1/16, A01K 61/10, A01K 61/95, G01N 33/18

(54) **DISPOSITIF POUR DÉTERMINER LE SUCCÈS DE LA REPRODUCTION DES POISSONS ET PROCÉDÉS UTILISANT LE-DIT DISPOSITIF**
VORRICHTUNG ZUR BESTIMMUNG DES FORTPFLANZUNGSERFOLGS VON FISCHEN UND VERFAHRENS ZUR VERWENDUNG DER VORRICHTUNG
DEVICE FOR DETERMINING THE SUCCESS OF REPRODUCTION OF FISHES AND METHODS USING SAID DEVICE

(30) Priorité: 17.12.2020 EP 20214870
(43) Date de publication de la demande: 22.06.2022
(73) Titulaire: Haute École Du Paysage D' Ingénierie Et D' Architecture (Hepia), 1202 Genève (CH)
(72) Inventeur: Rubin, Jean-Francois, 1131 Tolochenaz (CH); Robert-Charrue, Damien, 1131 Tolochenaz (CH)
(74) Mandataire: KATZAROV S.A.

(56) Documents cités:
- CN-U- 210 981 921
- US-A1- 2012 222 500
- US-A1- 2020 096 483

## Description

### DOMAINE TECHNIQUE DE L'INVENTION

L'invention concerne un dispositif à placer dans le lit d'un cours d'eau ou d'un plan d'eau pour l'étudier, pour déterminer le succès de la reproduction naturelle des poissons, notamment des salmonidés.

L'invention concerne également un procédé pour prélever un échantillon d'eau interstitielle d'un cours d'eau ou d'un plan d'eau.

L'invention concerne aussi un procédé pour déterminer la qualité biologique de l'eau d'un cours d'eau ou d'un plan d'eau.

### ETAT DE LA TECHNIQUE

La méthode classique pour déterminer la qualité de l'eau consiste à analyser divers paramètres physico-chimiques.

Le document US2012222500 décrit un système de surveillance utilisant un échantillonneur équipé d'une résine ou un autre adsorbant permettant l'absorption de contaminants et de polluants de l'eau ou de l'air. Le système comprend en outre des capteurs en temps réel qui détectent et transmettent des données physiques et/ou chimiques par télémétrie sans fil ou câblée et systèmes GPS.

Le document CN210981921 concerne un dispositif de détection basé sur l'Internet des Objets - IOT. Le système inclus une tige de corps avec un tuyau d'accumulation d'eau est agencé à l'intérieur, et un mécanisme de collecte agencé également dans le tuyau d'accumulation d'eau. Le mécanisme de collecte est agencé pour effectuer un échantillonnage fixe à différents niveaux d'eau.

Le document US2020096483 décrit un dispositif d'échantillonnage passif in situ comprenant trois échantillonneurs passifs et un dispositif d'élevage de graisses de poisson, dans lequel le dispositif d'élevage de poissons peut garantir l'environnement de survie du poisson dans le test à long terme, maintenir le taux de survie du poisson, et appliquer le dispositif à l'évaluation de sécurité de la qualité de l'eau de la source d'eau potable centralisée.

Ces approches ne permettent toutefois de qualifier que certains éléments à un endroit et un moment précis et n'est donc pas intégratrice de la situation à moyen ou long terme sur l'ensemble d'un territoire. Par ailleurs, selon les paramètres analysés, les déterminations peuvent être relativement onéreuses.

Les truites, comme les autres salmonidés, sont très sensibles à la qualité de leur habitat, donc aux variations de température et de qualité de l'eau. La dynamique de population des poissons témoigne donc de la santé des écosystèmes aquatiques.

Les jeunes salmonidés sont en effet particulièrement exigeants en matière de qualité d'eau qui elle dépend de l'état de l'ensemble du bassin versant. De fait, l'étude des premiers stades de vie permet d'identifier les problèmes d'une rivière à différentes échelles en fonction de divers facteurs biotiques et abiotiques.

Cependant, les méthodes existantes ne permettent pas d'étudier le cycle complet de développement des salmonidés allant de l'oeufs fraichement fécondé à l'émergence des alevins (egg-to-fry).

De plus, aucune méthode existante n'intègre l'étude des paramètres environnementaux clés physico-chimiques de l'eau interstitielle influençant directement le développement des organismes à chacun des stades de développement.

### OBJET DE L'INVENTION

Un objet de la présente invention est donc de résoudre, ou au moins de minimiser les inconvénients des méthodes de suivi et d'analyse de l'eau d'un cours d'eau ou d'un plan d'eau existantes décrites ci-dessus.

En particulier, le but de la présente invention est de permettre l'analyse de l'eau d'une rivière, en particulier l'eau interstitielle, pour obtenir des informations sur la qualité biologique de l'eau grâce à la bio-indication.

La bio-indication consiste à évaluer les conséquences des perturbations physico-chimiques sur les différentes espèces, à divers stades du cycle de vie, qui se trouvent dans le milieu. De cette manière, même une pollution diffuse peut être détectée. Les jeunes truites étant très exigeantes de ce point de vue, la réponse des populations, soumises à de telles pollutions, pourra être détectée. Ainsi, on utilise les jeunes stades de vie des poissons, en particulier les salmonidés comme sentinelles pour évaluer l'état écologique des écosystèmes aquatiques courants.

Dans un premier aspect, la présente invention concerne dispositif destiné à être placé dans le lit d'un cours d'eau ou d'un plan d'eau pour déterminer le succès de la reproduction des poissons, par exemple les salmonidés, le dispositif comprenant un fond et un couvercle positionné au regard dudit fond, autrement dit placé en face ou opposé au dit fond,
le fond et le couvercle étant liés de façon réversible par une face latérale s'étendant depuis le fond vers le couvercle selon un axe longitudinal du dispositif de façon à définir une chambre,
ladite face latérale comprenant des pores destinés à laisser passer l'eau du cours d'eau ou du plan d'eau dans la chambre,
le dispositif comprenant en outre plusieurs tubes creux s'étendant entre le fond et le couvercle selon l'axe longitudinal, chaque tube ayant une extrémité dite fermée qui est bouchée par le fond et une extrémité opposée dite ouverte traversant le couvercle et débouchant dans le cours d'eau ou le plan d'eau lorsque le dispositif est immergé,
chaque tube comprenant plusieurs ouvertures aménagées dans la paroi du tube destinées à laisser passer l'eau de la chambre dans le tube, de façon à créer une connexion fluidique entre l'extrémité ouverte desdits tubes et les pores de la face latérale via lesdites ouvertures.

La présente invention propose une méthode de mesure permettant d'évaluer la qualité des rivières grâce à l'étude des premiers stades de vie de poissons, en particulier des salmonidés. Durant la phase de l'oeuf à l'émergence (Egg-to-Fry), la survie des jeunes alevins dépend fortement de la qualité de l'eau superficielle et interstitielle (l'eau contenue dans le substrat). De ce fait, connaître le taux de survie des premiers stades de développement des salmonidés permet (1) d'adapter la gestion piscicole et (2) de déterminer la qualité des cours d'eau ou d'un plan d'eau et de leur bassin versant.

La réussite de la reproduction naturelle des poissons lithophiles dépend de nombreux paramètres, notamment des conditions écologiques présentes à l'intérieur même du substrat de ponte. Des paramètres physiques comme la température, la perméabilité et la granulométrie du substrat ou le taux de sédimentation en surface et à l'intérieur des frayères, sont essentiels, garantissant ou non le succès de la reproduction naturelle.

De plus, cette phase dans la vie des poissons est très peu étudiée en raison de la difficulté d'accès aux jeunes situés dans le gravier. En effet, même s'il est relativement aisé de compter les frayères dans un cours d'eau ou un plan d'eau, cela ne renseigne pas sur la survie des alevins durant les quelques mois qu'ils passeront cachés dans les interstices du gravier. Le comptage des jeunes lors de pêches électriques n'identifie que le nombre de poissons présents dans la rivière après plusieurs mois suivant leur naissance et en aucun cas le pourcentage de survie. Le nombre initial d'oeufs pondus étant inconnus.

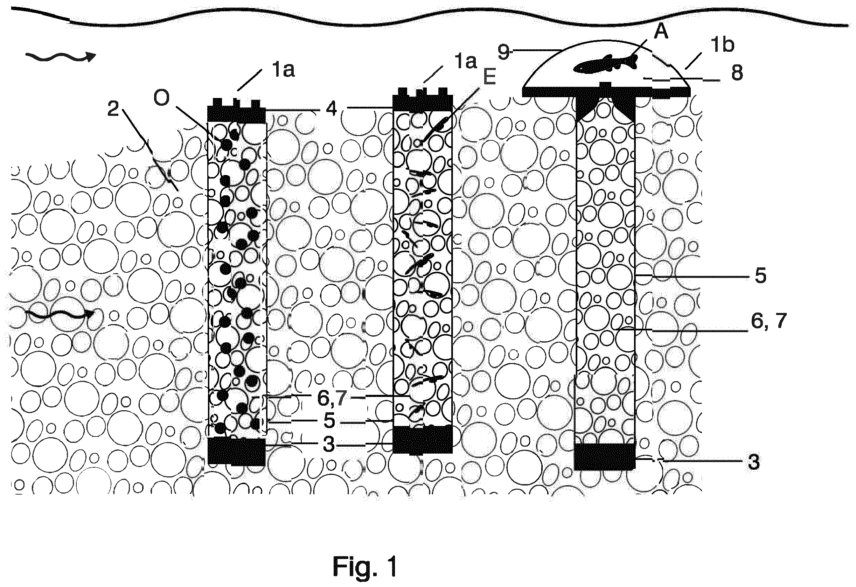

La présente invention propose de placer un dispositif dans le lit du cours d'eau ou d'un plan d'eau pour évaluer la qualité de l'eau de la rivière. Ce dispositif est enfoncé partiellement dans le lit pour ne laisser dépasser que le couvercle (figure 1). La chambre du dispositif est ainsi en contact avec l'eau s'écoulant dans le substrat graveleux (appelée eau interstitielle).

Chaque tube, également appelé tube de prélèvement, comporte plusieurs ouvertures aménagées dans la paroi du tube destinées à laisser passer l'eau de la chambre dans le tube, de façon à créer une connexion fluidique entre l'extrémité ouverte desdits tubes et les pores de la face latérale via lesdites ouvertures.

Selon un mode de réalisation préférée, les ouvertures sont réparties sur la longueur des tubes pour permettre un prélèvement d'eau à différentes profondeurs, par exemple le dispositif comprend trois tubes, un premier tube où les ouvertures sont proches du couvercle, un deuxième tube où les ouvertures sont proches du fond et un troisième tube où les ouvertures sont à mi-chemin entre le fond et le couvercle. Cette capacité de prélèvement à différentes profondeurs assure un suivi de l'eau interstitielle adéquat afin d'observer les variations dans le temps des paramètres clés influençant le développement des premiers stades de vie des salmonidés.

De préférence, chaque ouverture a un diamètre suffisamment petit pour empêcher le passage d'oeufs lorsqu'il y a des oeufs de poissons placés dans la chambre mais suffisamment grand pour permettre le passage de l'eau.

Ainsi, lorsque la chambre contient des oeufs de poisson d'une espèce déterminée, le diamètre des ouvertures est choisi pour bloquer l'entrée des oeufs dans lesdits tubes. De plus, les ouvertures sont définies et réparties afin de limiter le risque de colmatage des tubes de prélèvements et ainsi assurer la capacité de prélèvement de l'eau interstitielle en vue de son analyse.

De préférence, lesdites ouvertures ayant un diamètre compris entre environ 0.1 mm et 1.6 mm.

En particulier, chaque ouverture a un diamètre compris entre environ 0.50 mm et 1.5 mm, de préférence entre 1.0 et 1.5 mm, par exemple environ 1.0 mm, par exemple environ 15 mm

Selon un mode de réalisation préférée, le dispositif est de forme cylindrique, le fond et le couvercle définissant les bases dudit cylindre, lesdites bases étant connectées par la face latérale circulaire selon la hauteur dudit cylindre permettant de définir une chambre cylindrique.

Selon un mode de réalisation préférée, les ouvertures sont circulaires limitant ainsi les possibilités d'obturation par des éléments solides transitant dans les veines d'eau interstitielles ou par les oeufs.

De préférence, le dispositif comprend en outre un réducteur couplé sur le pourtour de la chambre à proximité du fond lorsque le fond est couplé à la face latérale,
le réducteur comprenant un plan incliné faisant face au couvercle lorsque le couvercle est couplé à la face latérale,
ledit réducteur permettant de faciliter l'écoulement du contenant de la chambre lorsque le fond de la chambre est ouvert, par exemple le réducteur est une bague.

Ainsi, les matériaux contenus dans la chambre ne se détériorent pas par frottement au passage de la bague. Cela permet également aux dit matériaux de ne pas rester bloqués sur le plan de la bague obligeant alors une intervention manuelle humaine sur le dispositif, notamment sur le matériel biologique.

De préférence, le réducteur est constitué d'un polymère, par exemple un plastique, notamment du POM.

Le dispositif selon la présente invention comprend plusieurs tubes creux s'étendant entre le fond et le couvercle selon l'axe longitudinal, chaque tube ayant une extrémité dite fermée qui est bouchée par le fond et une extrémité opposée dite ouverte traversant le couvercle et débouchant dans le cours d'eau ou le plan d'eau lorsque le dispositif est immergé.

De préférence, le dispositif comprend entre 2 et 10 tubes, de préférence entre 2 et 6 tubes, de préférence entre 2 et 4 tubes, par exemple 3 tubes.

Le nombre de tube est choisi selon les études menées sur les courants interstitiels des frayères à salmonidés et les besoins mécaniques structurant les dispositifs. Ainsi, entre 2 et 10 tubes permettent de répondre aux contraintes mécaniques fortes des dispositifs une fois implantés dans le substrat (cours d'eau ou plan d'eau par exemple). Entre 2 et 6 tubes permettent des prélèvements aux profondeurs clés démontrant des modifications de paramètres physico-chimiques dans le temps. Entre 2 et 4 tubes permettent une analyse satisfaisante biologiquement et économiquement afin de suivre finement l'évolution dans le temps des paramètres influençant la survie des premiers stades de vie des salmonidés.

Selon un mode de réalisation préférée, chaque tube comprend entre 1 et 20 ouvertures, de préférence entre 4 et 9 ouvertures, de préférence entre 5 et 7 ouvertures, par exemple 6 ouvertures.

Le nombre d'ouverture par tube est choisi selon la résistance mécanique des tubes ainsi que la quantité d'eau à prélever et le risque de colmatage du u dispositif, notamment des tubes. Entre 1 et 20 ouvertures garantissent la résistance face aux sollicitations mécaniques une fois les dispositifs dans le substrat. Entre 4 et 9 ouvertures permettent de prélever des échantillons de bonne qualité aux profondeurs clés. Entre 5 et 7 ouvertures garantissent les capacités de prélèvement ainsi que la qualité des échantillons d'eau tout en évitant le colmatage des tubes.

De préférence, au moins un desdits tubes comprend deux ouvertures opposées l'une à l'autre de part et d'autre dudit tube de façon à définir un canal traversant perpendiculaire à l'axe longitudinal du tube.

Dans la présente invention, il y a une connexion fluidique entre l'eau présente à l'extérieure du dispositif lorsque le dispositif est immergé et celle à l'intérieur de la chambre. L'eau peut s'écouler à travers le dispositif et entrer dans le dispositif depuis l'extérieur du dispositif (lit du cours d'eau par exemple) par l'extrémité ouverte du tube puis à travers les ouvertures du tube et les pores de la paroi latérale pour ressortir du dispositif. Cette circulation est possible dans les deux sens, de l'extérieur vers intérieur du dispositif et vice versa. Cette circulation ou connexion fluidique est particulièrement avantageuse car elle permet de garantir le développement du matériel biologique contenu dans le dispositif, notamment dans la chambre, ce qui généralement la finalité de l'étude. De plus, ces échanges de fluides miment le comportement naturel des eaux interstitielles assurant ainsi la qualité des échantillons prélevés au fil du temps.

La paroi latérale comprend des pores qui permettent un écoulement de l'eau à travers la paroi. De préférence les pores retiennent les contenants de la chambre sauf l'eau. Par exemple les pores ont un diamètre compris entre 60 mm et 1 mm afin de garantir le maintien du substrat dans les dispositifs. Entre 20 mm et 1 mm afin d'assurer une hétérogénéité du substrat mimant le cycle naturel. Entre 8 mm et 1 mm garantissant les écoulements fluidiques malgré le colmatage par les particules fines. Entre 3 mm et 1 mm afin de garantir le maintien des oeufs de salmonidés dans les dispositifs. Par exemple, des pores de 2 mm.

Selon un mode de réalisation préféré, le fond et/ou le couvercle comprend des moyens de couplage réversible pour solidariser le fond et/ou le couvercle avec la face, par exemple par vissage. Cette caractéristique permet de garantir aux dispositifs dans le substrat leur résistance aux fortes contraintes mécaniques. De plus, le vissage permet de réparer ou modifier les dispositifs en fonction de l'utilisation, notamment selon le type d'étude menée.

De préférence, le fond comprend des moyens de préhension, par exemple une denture annulaire ou des encoches, pour faciliter l'attachement et le détachement du fond de la face latérale.

De préférence, le dispositif comprend une capsule, par exemple un dôme, couplée sur le couvercle et définissant chambre externe entre la capsule et ledit couvercle, le couvercle comprenant un canal central pour assurer une connexion, par exemple une connexion fluidique, entre la chambre et la chambre externe. Cette ouverture permet de garantir la libre circulation des salmonidés émergents. Ainsi leurs comportements naturels sont possibles garantissant de facto le passage du dernier stade de vie de la larve au premier stade de vie de l'alevin.

Selon un mode de réalisation préférée, le dispositif est une boite à émergence ou une boite à éclosion. La différence entre une boite à émergence et une boite à éclosion réside dans le fait que la boite à émergence comprend une capsule appelée dôme qui est couplée sur le couvercle de la boite de manière mécanique. Le dôme est destiné à accueillir les alevins. Une boite à émergence est donc une boite à éclosion sur laquelle on ajoute un dôme.

La présente invention permet de faciliter les prélèvements de cette eau interstitielle sans créer de perturbations dans les frayères (qui biaiseraient de facto les résultats) ce qui est novateur par rapport aux méthodes traditionnelles.

La présente invention peut être utilisée pour prélever un échantillon d'eau, en particulier d'eau interstitielle un échantillon d'eau interstitielle d'un cours d'eau ou d'un plan d'eau, le procédé comprenant, de préférence se déroulant comme suit :
- Fournir un dispositif selon l'invention;
- Ouvrir le couvercle et introduire dans la chambre plusieurs constituants prélevés dans le lit du cours d'eau ou du plan d'eau, par exemple du gravier;
- Fermer le couvercle;
- Placer le dispositif dans le lit du cours d'eau ou du plan d'eau de façon à ce que le couvercle affleure à la surface du lit du cours d'eau ou du plan d'eau, la chambre étant enfoncée dans le lit pour que l'eau interstitielle pénètre dans la chambre à travers les pores de la face latérale;
- Prélever un échantillon d'eau interstitielle par l'extrémité ouverte d'un des tubes.
Ce mode de prélèvement permet de garantir la qualité des échantillons. Cela permet le suivi de leur évolution naturelle dans le temps, durant la phase de développement de l'oeuf à l'émergence des salmonidés.

Un échantillon prélevé par une méthode selon la présente invention, peut être ensuite analysé par des techniques classiques physico chimiques existantes, l'originalité de la présente invention résidant dans le mode de prélèvement.

De plus, lorsque des oeufs de poisson sont placés dans la chambre, il est possible d'évaluer indirectement la qualité de l'eau, (l'eau interstitielle), en suivant leur survie et leur développement.

Ainsi, la présente invention concerne un procédé pour déterminer la qualité de l'eau de cours d'eau ou de plans d'eau, de préférence à l'aide d'indicateurs biologiques, le procédé comprenant, de préférence se déroulant comme suit :
- Fournir un dispositif selon la présente invention;
- Ouvrir le couvercle;
- Introduire dans la chambre plusieurs constituants prélevés dans le lit du cours d'eau ou du plan d'eau, par exemple du gravier, et plusieurs oeufs de poissons, par exemple les salmonidés,
- Fermer le couvercle;
- Placer le dispositif dans le lit du cours d'eau ou du plan d'eau de façon à ce que le couvercle affleure à la surface du lit du cours d'eau ou du plan d'eau, la chambre étant enfoncée dans le lit pour que l'eau interstitielle pénètre dans la chambre à travers les pores de la face latérale;
- Suivre le développement des oeufs de la chambre et en fonction de ce développement, déterminer la qualité de l'eau du cours d'eau ou du plan d'eau, en particulier de l'eau interstitielle ;
Ce mode de suivi de l'écosystème aquatique par des espèces (ici salmonidés) qui se développent dans le dispositif et qui ont un rôle de sentinelle permet de comprendre l'état écologique général des systèmes, notamment des éco systèmes, ainsi que leur évolution naturelle dans le temps. Ces dispositifs permettent également de comprendre les facteurs limitants le développement des salmonidés de l'oeuf à l'émergence en lien avec les activités humaines ou naturelles dans les bassins versants.

De préférence, le procédé comprend, de préférence se déroulant comme suit :
- un échantillon d'eau interstitielle est prélevé à intervalle déterminée, par exemple régulier, par l'extrémité ouverte d'un des tubes;
- l'échantillon est analysé pour permettre la détermination de la qualité de l'eau, par exemple la présence d'une source de pollution.

Cette caractéristique permet de suivre les paramètres physico-chimiques limitant le développement des salmonidés de l'oeufs à l'émergence. Notamment les modifications des paramètres des eaux interstitielles dans le temps comme la baisse de qualités des facteurs environnementaux clés ou la présence de polluants.

Le dispositif comprend en outre divers composants en connexion avec les fluides sous graviers, ce qui permet de prélever de l'eau interstitielle. Cette connexion implique notamment :
- la face latérale du dispositif poreuse;
- les ouvertures des tubes;
- l'extrémité ouverte des tubes.

L'eau pénètre dans la chambre via les pores, puis entre dans le tube par l'intermédiaire des ouvertures. Il est ainsi possible de prélever de l'eau interstitielle.

Les modes de réalisation décrits pour le dispositif selon la présente invention s'appliquent également aux procédés selon l'invention mutatis mutandis et vice versa.

### BRÈVE DESCRIPTION DES FIGURES

D'autres avantages, buts et caractéristiques particulières de l'invention ressortiront de la description non limitative qui suit d'au moins un mode de réalisation particulier du dispositif et du procédé objets de la présente invention, en regard des dessins annexés, dans lesquels
- La figure 1 illustre dispositif immergé dans le lit d'un cours d'eau;
- Les figures 2 à 5 représentent une boite à éclosion qui est un exemple de dispositif selon la présente invention;
- Les figures 6 à 7 représentent une boite à émergence qui est un exemple de dispositif selon la présente invention;

### DESCRIPTION D'EXEMPLES DE RÉALISATION DE L'INVENTION

La présente description est donnée à titre non limitatif, chaque caractéristique d'un mode de réalisation pouvant être combinée à toute autre caractéristique de tout autre mode de réalisation de manière avantageuse.

La figure 1 représente un dispositif selon la présente invention, dans cet exemple une boite à éclosion 1a et une boite à émergence 1b placées dans le lit d'un cours d'eau 2.

La boite 1a,b comprend un fond 3 et un couvercle 4 opposé au fond 3. Le fond 3 et le couvercle 4 étant liés de façon réversible par une face latérale 5 s'étendant depuis le fond 3 vers le couvercle 4 selon un axe longitudinal L du dispositif de façon à définir une chambre 6. La face latérale 5 comprend des pores 7 destinés à laisser passer l'eau du cours d'eau 2 dans la chambre 6.

La boite 1 est immergée dans le lit du cours d'eau 2 de façon à ce que le couvercle 4 affleure à la surface du lit du cours d'eau 2.

La chambre 5 est enfoncée dans le lit 2 pour que l'eau interstitielle pénètre dans la chambre 6 à travers les pores 7 de la face latérale 5.

La boite 1 est destinée à permettre le développement d'alvins A selon le procédé suivant :
- Étape 1 (bas de la chambre 6 - coté fond 3) : les oeufs O se développent durant près de quatorze semaines à l'abri dans le gravier de la chambre 6 avant d'éclore;
- Étape 2 (milieu de la chambre 6 - mi chemin entre fond 3 et couvercle 4) : les alevins A vésiculés restent environ quatre semaines dans le gravier de la chambre 6 pour se développer en vivant sur leur réserve de nourriture et atteindre le stade de larve E;
- Étape 3 (haut de la chambre 6 - coté couvercle 4): les alevins A sortent du gravier de la chambre 6 pour attendre une chambre interne 8 délimitées par un dôme 9; les alevins A sont bloqués dans le dôme 9 ce qui permet ainsi le comptage.

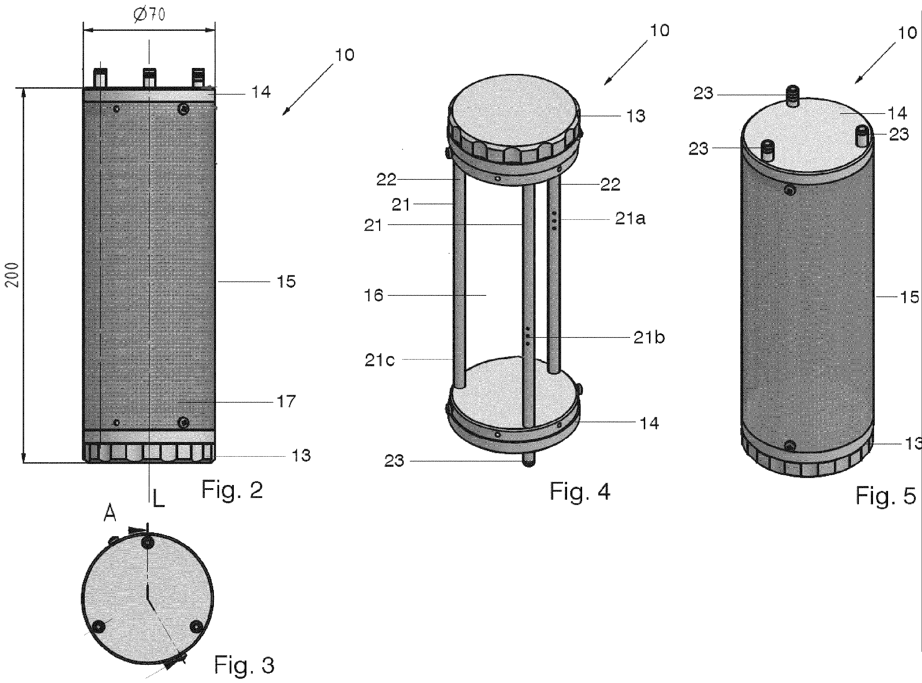

Les figures 2 à 5 représentent une boite à éclosion 10 qui est un exemple de dispositif selon la présente invention.

La boite 10 comprend un fond 13 et un couvercle 14 opposé au fond 13. Le fond 13 et le couvercle 14 étant liés de façon réversible par une face latérale 15 s'étendant depuis le fond 13 vers le couvercle 14 selon un axe longitudinal L du dispositif de façon à définir une chambre 16. La face latérale 15 comprend des pores 17 destinés à laisser passer l'eau du cours d'eau dans la chambre 16. La boite 10 est de forme cylindrique, le fond 13 et le couvercle 14 définissant les bases dudit cylindre, lesdites bases étant connectées par la face latérale circulaire 15 selon la hauteur dudit cylindre permettant de définir une chambre cylindrique 16.

La boite 10 comprend ici trois tubes creux 21 s'étendant entre le fond 13 et le couvercle 14 selon l'axe longitudinal L. Chaque tube 21 a une extrémité dite fermée 22 qui est bouchée par le fond 13 et une extrémité opposée dite ouverte 23 traversant le couvercle 14 et débouchant dans le cours d'eau ou le plan d'eau lorsque la boite 10 est immergé.

Chaque tube 21 présente ici six ouvertures 24 aménagées dans la paroi du tube 21 destinées à laisser passer l'eau de la chambre 16 dans le tube 21, de façon à créer une connexion fluidique entre l'extrémité ouverte 23 desdits tubes et les pores 17 de la face latérale 15 via lesdites ouvertures 24.

Dans cet exemple, deux ouvertures 24 sont à chaque fois opposées l'une à l'autre de part et d'autre dudit tube 21 de façon à définir un canal traversant perpendiculaire à l'axe longitudinal du tube. Les ouvertures 24 sont circulaires. Chaque ouverture 24 présente un diamètre de 1.5 mm.

Dans cet exemple, les ouvertures 24 sont répartis sur la longueur des tubes 21 pour permettre un prélèvement d'eau à différentes profondeurs. Ici un premier tube 21a ou les ouvertures 24 proche du couvercle 14, un deuxième tube 21 c ou les ouvertures 24 sont proches du fond 23 et un troisième tube 21b ou les ouvertures 24 sont à mi-chemin entre le fond 23 et le couvercle 24.

Avantageusement, il est possible d'effectuer des prélèvements d'eau interstitielle en utilisant l'extrémité ouverte 23 qui passe à travers le couvercle 14. Un échantillon d'eau interstitielle est prélevé à intervalle déterminée, par exemple régulier, par l'extrémité ouverte 24 d'un des tubes 21. Puis l'échantillon est analysé pour permettre la détermination de la qualité de l'eau, par exemple la présence d'une source de pollution.

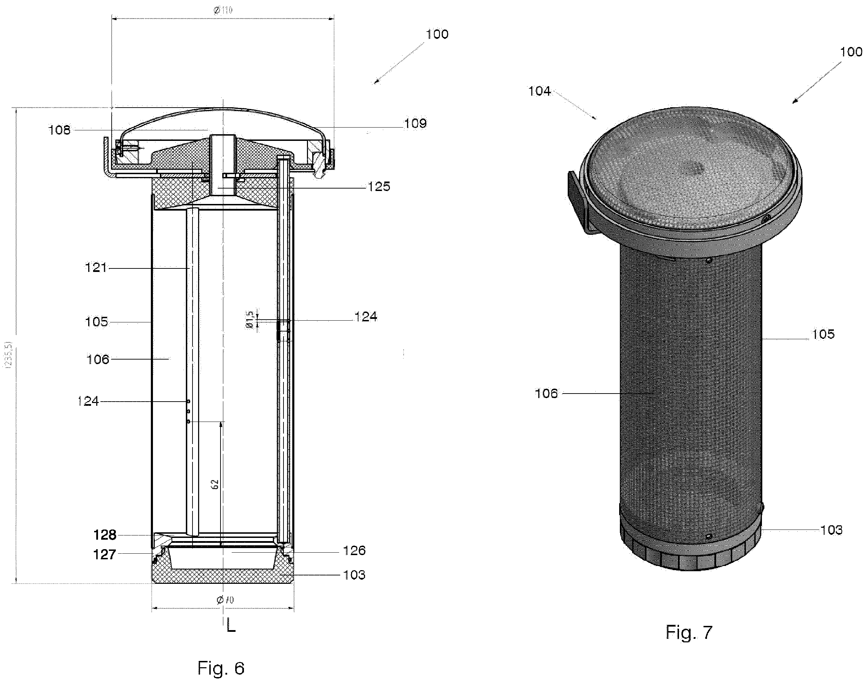

Les figures 6 à 7 représentent une boite à émergence 100 qui est un exemple de dispositif selon la présente invention.

La boite 100 comprend un fond 103 et un couvercle 104 opposé au fond 103. Le fond 103 et le couvercle 104 étant liés de façon réversible par une face latérale 105 s'étendant depuis le fond 103 vers le couvercle 104 selon un axe longitudinal L du dispositif de façon à définir une chambre 106. La face latérale 105 comprend des pores 107 destinés à laisser passer l'eau du cours d'eau dans la chambre 106. La boite 100 est de forme cylindrique, le fond 113 et le couvercle 114 définissant les bases dudit cylindre, lesdites bases étant connectées par la face latérale circulaire 115 selon la hauteur dudit cylindre permettant de définir une chambre cylindrique 116.

La boite 100 comprend ici trois tubes creux 121 s'étendant entre le fond 103 et le couvercle 104 selon l'axe longitudinal L. Chaque tube 121 a une extrémité dite fermée 122 qui est bouchée par le fond 113 et une extrémité opposée dite ouverte 123 traversant le couvercle 114 et débouchant dans le cours d'eau ou le plan d'eau lorsque la boite 100 est immergé.

Chaque tube 121 présente ici six ouvertures 124 aménagées dans la paroi du tube 121 destinées à laisser passer l'eau de la chambre 116 dans le tube 121, de façon à créer une connexion fluidique entre l'extrémité ouverte 123 desdits tubes et les pores 17 de la face latérale 115 via lesdites ouvertures 124.

La boite à émergence 100 comprend un dôme 109, couplée sur le couvercle 113 et définissant une chambre externe 108 entre le dôme 109 et ledit couvercle 113. Le couvercle comprend un canal central 126 pour assurer une connexion fluidique entre la chambre 116 et la chambre externe 108.

Le dôme est destiné à accueillir les alevins au niveau de la chambre interne comme représenté sur la figure 1.

Dans cet exemple, la boite 100 comprend en outre un réducteur sous la forme d'une bague circulaire 127. La bague 127 couplée sur le pourtour de la chambre 116 à proximité du fond 113.

La bague 127 comprenant un plan incliné 128 faisant face au couvercle 113 ce qui permet de faciliter l'écoulement du contenant de la chambre 116 lorsque le fond de la chambre 116 est ouvert, par exemple pour vider la boite 100.

Les boites 10 et 100 comprennent chacune des moyens de préhension sur le contour du couvercle 13,103, ici denture annulaire pour faciliter l'attachement et le détachement du fond 13,103 de la face latérale 15,115, par exemple pour vider la chambre 16,116.

Avantageusement, la boite 100 peut être utilisée pour déterminer la qualité de l'eau de cours d'eau ou de plans d'eau en suivant le développement des alevins. En fonction de ce développement, il est possible de déterminer la qualité de l'eau du cours d'eau ou du plan d'eau, en particulier de l'eau interstitielle.

### Numéro de référence

- 1: Boite à émergence
- 2: Lit de la rivière
- 3: Fond
- 4: Couvercle
- 5: Face latérale
- 6: Chambre
- 7: Pore de la face latérale
- 8: Chambre interne
- 9: Dôme
- A: Alevins
- E: Larve éclose
- O: Oeufs

- 10: Boite à éclosion
- 13: Fond
- 14: Couvercle
- 15: Face latérale
- 16: Chambre
- 17: Pore de la face latérale
- 21: Tube
- 22: Extrémité fermée
- 23: Extrémité ouverte
- 24: Ouvertures
- 25: Denture annulaire

- 100: Boite à émergence
- 103: Fond
- 104: Couvercle
- 105: Face latérale
- 106: Chambre
- 107: Pore de la face latérale
- 108: Chambre interne
- 109: Dôme
- 121: Tube
- 122: Extrémité fermée
- 123: Extrémité ouverte
- 124: Ouvertures
- 125: Denture
- 126: Canal central
- 127: Bague circulaire
- 128: Plan incliné

## Revendications

1. Dispositif (1,10,100) destiné à être placé dans le lit d'un cours d'eau (2) ou d'un plan d'eau pour déterminer le succès de la reproduction des poissons, par exemple les salmonidés, le dispositif (1,10,100) comprenant
un fond (3,13,103) et un couvercle (4,14,104) positionné au regard dudit fond (3,13,103),
le fond (3,13,103) et le couvercle (4,14,104) étant liés de façon réversible par une face latérale (5,15,105) s'étendant depuis le fond (3,13,103) vers le couvercle (4,14,104) selon un axe longitudinal (L) du dispositif (1,10,100) de façon à définir une chambre (6,16,106),
ladite face latérale (5,15,105) comprenant des pores (7,17,127) destinés à laisser passer l'eau du cours d'eau (2) ou du plan d'eau dans la chambre (6,16,116),
le dispositif (1,10,100) comprenant en outre plusieurs tubes (21,121) creux s'étendant entre le fond (3,13,103) et le couvercle (4,14,104) selon l'axe longitudinal (L), chaque tube (21,121) ayant une extrémité dite fermée (22,122) qui est bouchée par le fond (3,13,103) et une extrémité opposée dite ouverte (23,123) traversant le couvercle (4,14,104) et débouchant dans le cours d'eau (2) ou le plan d'eau lorsque le dispositif (1,10,100) est immergé,
chaque tube (21,121) comprenant plusieurs ouvertures (24,124) aménagées dans la paroi du tube (21,121) destinées à laisser passer l'eau de la chambre (6,16,116) dans le tube (21,121), de façon à créer une connexion fluidique entre l'extrémité ouverte desdits tubes (21,121) et les pores (7,17,127) de la face latérale (5,15,105) via lesdites ouvertures (24,124).

2. Dispositif (1,10,100) selon la revendication 1, dans lequel les ouvertures (24,124) sont répartis sur la longueur des tubes (21,121) pour permettre un prélèvement d'eau à différentes profondeurs, par exemple le dispositif (1,10,100) comprend trois tubes (1,10,100), un premier tube (21a) où les ouvertures (24) sont proches du couvercle (14), un deuxième tube (21c) où les ouvertures (24) sont proches du fond (13) et un troisième tube (21b) où les ouvertures (24) sont à mi-chemin entre le fond (13) et le couvercle (14).

3. Dispositif (1,10,100) selon l'une des revendications précédentes, dans lequel chaque ouverture (24,124) a un diamètre suffisamment petit pour empêcher le passage d'oeufs lorsqu'il y a des oeufs de poissons placés dans la chambre (6,16,116) mais suffisamment grand pour permettre le passage de l'eau, lesdites ouvertures ayant un diamètre compris entre environ 0.1 mm et 1.6 mm, de préférence chaque ouverture a un diamètre compris entre environ 0.50 mm et 1.5 mm, plus préférablement entre 1.0 et 1.5 mm, par exemple environ 1.0 mm ou environ 1.5 mm.

4. Dispositif (1,10,100) selon l'une des revendications précédentes, dans lequel le dispositif (1,10,100) est de forme cylindrique, le fond (3,13,113) et le couvercle (4,14,114) définissant les bases dudit cylindre, lesdites bases étant connectées par la face latérale (5,15,115) circulaire selon la hauteur dudit cylindre permettant de définir une chambre cylindrique.

5. Dispositif (1,10,100) selon l'une des revendications précédentes, dans lequel le dispositif (1,10,100) comprend en outre un réducteur couplé sur le pourtour de la chambre (6,16,116) à proximité du fond (3,13,113) lorsque le fond (3,13,113) est couplé à la face latérale (5,15,115),
le réducteur comprenant un plan incliné (126) faisant face au couvercle (4,14,114) lorsque le couvercle (4,14,114) est couplé à la face latérale (5,15,115),
ledit réducteur permettant de faciliter l'écoulement du contenant de la chambre (6,16,116) lorsque le fond (3,13,113) de la chambre (6,16,116) est ouvert, par exemple le réducteur est une bague (127).

6. Dispositif (1,10,100) selon l'une des revendications précédentes, dans lequel le dispositif (1,10,100) comprend entre 2 et 10 tubes (21,121), de préférence entre 2 et 6 tubes (21,121), de préférence entre 2 et 4 tubes (21,121), par exemple 3 tubes (21,121).

7. Dispositif (1,10,100) selon l'une des revendications précédentes, dans lequel chaque tube (21,121) comprend entre 1 et 20 ouvertures (24,124), de préférence entre 4 et 9 ouvertures (24,124), de préférence entre 5 et 7 ouvertures (24,124), par exemple 6 ouvertures (24,124).

8. Dispositif (1,10,100) selon l'une des revendications précédentes, dans lequel au moins un desdits tubes (21,121) comprend deux ouvertures (24,124) opposées l'une à l'autre de part et d'autre dudit tube de façon à définir un canal traversant perpendiculaire à l'axe longitudinal du tube (L).

9. Dispositif (1,10,100) selon l'une des revendications précédentes, dans lequel chaque ouverture (24,124) a un diamètre compris entre environ 0.50 mm et 1.5 mm, de préférence entre 1.0 et 1.5 mm, par exemple environ 1.0 mm ou environ 1.5 mm

10. Dispositif (1,10,100) selon l'une des revendications précédentes, dans lequel le fond (3,13,113) et/ou le couvercle (4,14,114) comprend des moyens de couplage réversible pour solidariser le fond et/ou le couvercle avec la face, par exemple par vissage.

11. Dispositif (1,10,100) selon l'une des revendications précédentes, dans lequel le dispositif (1,10,100) comprend une capsule, par exemple un dôme (9,109), couplée sur le couvercle (4,114) et définissant chambre externe (8,108) entre la capsule et ledit couvercle (4,114), le couvercle (4,114) comprenant un canal central (126) pour assurer une connexion fluidique entre la chambre (116) et la chambre externe (108).

12. Procédé pour prélever un échantillon d'eau interstitielle d'un cours d'eau ou d'un plan d'eau, le procédé comprenant :
- Fournir un dispositif (10) selon l'une des revendications 1 à 10;
- Ouvrir le couvercle (14) et introduire dans la chambre (16) plusieurs constituants prélevés dans le lit du cours d'eau ou du plan d'eau, par exemple du gravier;
- Fermer le couvercle (14);
- Placer le dispositif (10) dans le lit du cours d'eau ou du plan d'eau de façon à ce que le couvercle (14) affleure à la surface du lit du cours d'eau ou du plan d'eau, la chambre (16) étant enfoncée dans le lit pour que l'eau interstitielle pénètre dans la chambre (16) à travers les pores (17) de la face latérale (15);
- Prélever un échantillon d'eau interstitielle par l'extrémité ouverte (23) d'un des tubes (21).

13. Procédé pour déterminer la qualité de l'eau de cours d'eau ou de plans d'eau, de préférence à l'aide d'indicateurs biologiques, le procédé comprenant:
- Fournir un dispositif (1,100) selon l'une des revendications 1 à 10;
- Ouvrir le couvercle (4,114);
- Introduire dans la chambre (16,116) plusieurs constituants prélevés dans le lit du cours d'eau ou du plan d'eau, par exemple du gravier, et plusieurs oeufs de poissons, par exemple les salmonidés,
- Fermer le couvercle (14,114);
- Placer le dispositif (14,114) dans le lit du cours d'eau ou du plan d'eau de façon à ce que le couvercle (14,114) affleure à la surface du lit du cours d'eau ou du plan d'eau, la chambre (16,116) étant enfoncée dans le lit pour que l'eau interstitielle pénètre dans la chambre (16,116) à travers les pores (17,117) de la face latérale (15,115);
- Suivre le développement des oeufs de la chambre (16,116) et en fonction de ce développement, déterminer la qualité de l'eau du cours d'eau ou du plan d'eau, en particulier de l'eau interstitielle ;

14. Procédé selon la revendication précédente, dans lequel,
- un échantillon d'eau interstitielle est prélevé à intervalle déterminée, par exemple régulier, par l'extrémité ouverte (24,124) d'un des tubes (21,121);
- l'échantillon est analysé pour permettre la détermination de la qualité de l'eau, par exemple la présence d'une source de pollution.

## Patentansprüche

1. Vorrichtung (1, 10, 100), die dazu bestimmt ist, im Bett eines Fließgewässers (2) oder eines Stillgewässers platziert zu werden, um den Erfolg der Fortpflanzung von Fischen, zum Beispiel Salmoniden, zu bestimmen, wobei die Vorrichtung (1, 10, 100) umfasst:
einen Boden (3, 13, 103) und einen Deckel (4, 14, 104), der gegenüber dem Boden (3, 13, 103) angeordnet ist,
wobei der Boden (3, 13, 103) und der Deckel (4, 14, 104) auf reversible Weise durch eine Seitenfläche (5, 15, 105) verbunden sind, die sich von dem Boden (3, 13, 103) aus zu dem Deckel (4, 14, 104) hin entlang einer Längsachse (L) der Vorrichtung (1, 10, 100) erstreckt, so dass eine Kammer (6, 16, 106) definiert wird,
wobei die Seitenfläche (5, 15, 105) Poren (7, 17, 127) umfasst, die dazu bestimmt sind, das Wasser des Fließgewässers (2) oder des Stillgewässers in die Kammer (6, 16, 116) durchtreten zu lassen,
wobei die Vorrichtung (1, 10, 100) ferner mehrere hohle Röhren (21, 121) umfasst, die sich zwischen dem Boden (3, 13, 103) und dem Deckel (4, 14, 104) entlang der Längsachse (L) erstrecken, wobei jede Röhre (21, 121) ein sogenanntes geschlossenes Ende (22, 122), das durch den Boden (3, 13, 103) verschlossen wird, und ein entgegengesetztes, sogenanntes offenes Ende (23, 123), das den Deckel (4, 14, 104) durchquert und in dem Fließgewässer (2) oder dem Stillgewässer mündet, wenn die Vorrichtung (1, 10, 100) untergetaucht ist, aufweist,
wobei jede Röhre (21, 121) mehrere Öffnungen (24, 124) umfasst, die in der Wand der Röhre (21, 121) ausgebildet sind und dazu bestimmt sind, das Wasser der Kammer (6, 16, 116) in die Röhre (21, 121) durchtreten zu lassen, so dass eine Fluidverbindung zwischen dem offenen Ende der Röhren (21, 121) und den Poren (7, 17, 127) der Seitenfläche (5, 15, 105) über die Öffnungen (24, 124) geschaffen wird.

2. Vorrichtung (1, 10, 100) nach Anspruch 1, wobei die Öffnungen (24, 124) über die Länge der Röhren (21, 121) verteilt sind, um eine Wasserentnahme in verschiedenen Tiefen zu ermöglichen, wobei die Vorrichtung (1, 10, 100) zum Beispiel drei Röhren (1, 10, 100) umfasst, eine erste Röhre (21a), bei der die Öffnungen (24) nahe dem Deckel (14) sind, eine zweite Röhre (21c), bei der die Öffnungen (24) nahe dem Boden (13) sind, und eine dritte Röhre (21b), bei der die Öffnungen (24) auf halbem Weg zwischen dem Boden (13) und dem Deckel (14) sind.

3. Vorrichtung (1, 10, 100) nach einem der vorhergehenden Ansprüche, wobei jede Öffnung (24, 124) einen Durchmesser aufweist, der ausreichend klein ist, um das Durchtreten von Eiern, wenn Fischeier in der Kammer (6, 16, 116) platziert sind, zu verhindern, aber ausreichend groß ist, um das Durchtreten des Wassers zu ermöglichen, wobei die Öffnungen einen Durchmesser zwischen etwa 0,1 mm und 1,6 mm aufweisen, jede Öffnung bevorzugt einen Durchmesser zwischen etwa 0,50 mm und 1,5 mm, noch bevorzugter zwischen 1,0 und 1,5 mm, beispielsweise von etwa 1,0 mm oder etwa 1,5 mm, aufweist.

4. Vorrichtung (1, 10, 100) nach einem der vorhergehenden Ansprüche, wobei die Vorrichtung (1, 10, 100) von zylindrischer Form ist, wobei der Boden (3, 13, 113) und der Deckel (4, 14, 114) die Grundflächen des Zylinders definieren, wobei die Grundflächen durch die kreisförmige Seitenfläche (5, 15, 115) entlang der Höhe des Zylinders verbunden sind, was es ermöglicht, eine zylindrische Kammer zu definieren.

5. Vorrichtung (1, 10, 100) nach einem der vorhergehenden Ansprüche, wobei die Vorrichtung (1, 10, 100) ferner ein Reduzierstück umfasst, das an den Umfang der Kammer (6, 16, 116) in der Nähe des Bodens (3, 13, 113) gekoppelt ist, wenn der Boden (3, 13, 113) an die Seitenfläche (5, 15, 115) gekoppelt ist,
wobei das Reduzierstück eine schräge Ebene (126) umfasst, die dem Deckel (4, 14, 114) zugewandt ist, wenn der Deckel (4, 14, 114) an die Seitenfläche (5, 15, 115) gekoppelt ist,
wobei das Reduzierstück es ermöglicht, das Fließen des Inhalt der Kammer (6, 16, 116) zu erleichtern, wenn der Boden (3, 13, 113) der Kammer (6, 16, 116) offen ist, wobei das Reduzierstück zum Beispiel ein Ring (127) ist.

6. Vorrichtung (1, 10, 100) nach einem der vorhergehenden Ansprüche, wobei die Vorrichtung (1, 10, 100) zwischen 2 und 10 Röhren (21, 121), bevorzugt zwischen 2 und 6 Röhren (21, 121), bevorzugt zwischen 2 und 4 Röhren (21, 121), zum Beispiel 3 Röhren (21, 121), umfasst.

7. Vorrichtung (1, 10, 100) nach einem der vorhergehenden Ansprüche, wobei jede Röhre (21, 121) zwischen 1 und 20 Öffnungen (24, 124), bevorzugt zwischen 4 und 9 Öffnungen (24, 124), bevorzugt zwischen 5 und 7 Öffnungen (24, 124), zum Beispiel 6 Öffnungen (24, 124), umfasst.

8. Vorrichtung (1, 10, 100) nach einem der vorhergehenden Ansprüche, wobei mindestens eine der Röhren (21, 121) zwei Öffnungen (24, 124) umfasst, die entgegengesetzt zueinander beidseits der Röhre sind, so dass ein durchgehender Kanal senkrecht zu der Längsachse der Röhre (L) definiert wird.

9. Vorrichtung (1, 10, 100) nach einem der vorhergehenden Ansprüche, wobei jede Öffnung (24, 124) einen Durchmesser zwischen etwa 0,50 mm und 1,5 mm, bevorzugt zwischen 1,0 und 1,5 mm, beispielsweise von etwa 1,0 mm oder etwa 1,5 mm, aufweist.

10. Vorrichtung (1, 10, 100) nach einem der vorhergehenden Ansprüche, wobei der Boden (3, 13, 113) und/oder der Deckel (4, 14, 114) Mittel zur reversiblen Kopplung umfasst, um den Boden und/oder den Deckel mit der Seite zu verbinden, zum Beispiel durch Schrauben.

11. Vorrichtung (1, 10, 100) nach einem der vorhergehenden Ansprüche, wobei die Vorrichtung (1, 10, 100) eine Kapsel, zum Beispiel eine Kuppel (9, 109), umfasst, die an den Deckel (4, 114) gekoppelt ist und eine äußere Kammer (8, 108) zwischen der Kapsel und dem Deckel (4, 114) definiert, wobei der Deckel (4, 114) einen zentralen Kanal (126) umfasst, um eine Fluidverbindung zwischen der Kammer (116) und der äußeren Kammer (108) zu gewährleisten.

12. Verfahren zum Entnehmen einer Porenwasserprobe eines Fließgewässers oder eines Stillgewässers, wobei das Verfahren umfasst:
- Bereitstellen einer Vorrichtung (10) nach einem der Ansprüche 1 bis 10;
- Öffnen des Deckels (14) und Einführen mehrerer Bestandteile, die aus dem Bett des Fließgewässers oder des Stillgewässers entnommen wurden, zum Beispiel Kies, in die Kammer (16);
- Schließen des Deckels (14);
- Platzieren der Vorrichtung (10) in dem Bett des Fließgewässers oder des Stillgewässers, so dass der Deckel (14) bündig mit der Oberfläche des Betts des Fließgewässers oder des Stillgewässers ist, wobei die Kammer (16) in das Bett eingedrückt ist, damit das Porenwasser durch die Poren (17) der Seitenfläche (15) in die Kammer (16) eindringt;
- Entnehmen einer Porenwasserprobe durch das offene Ende (23) einer der Röhren (21).

13. Verfahren zum Bestimmen der Wasserqualität von Fließgewässern oder von Stillgewässern, bevorzugt mithilfe von biologischen Indikatoren, wobei das Verfahren umfasst:
- Bereitstellen einer Vorrichtung (1, 100) nach einem der Ansprüche 1 bis 10;
- Öffnen des Deckels (4, 114);
- Einführen mehrerer Bestandteile, die aus dem Bett des Fließgewässers oder des Stillgewässers entnommen wurden, zum Beispiel Kies, und von mehreren Fischeiern, zum Beispiel Salmoniden, in die Kammer (16, 116),
- Schließen des Deckels (14, 114);
- Platzieren der Vorrichtung (14, 114) in dem Bett des Fließgewässers oder des Stillgewässers, so dass der Deckel (14, 114) bündig mit der Oberfläche des Betts des Fließgewässers oder des Stillgewässers ist, wobei die Kammer (16, 116) in das Bett eingedrückt ist, damit das Porenwasser durch die Poren (17, 117) der Seitenfläche (15, 115) in die Kammer (16, 116) eindringt;
- Beobachten der Entwicklung der Eier der Kammer (16, 116) und in Abhängigkeit von dieser Entwicklung Bestimmen der Wasserqualität des Fließgewässers oder des Stillgewässers, insbesondere des Porenwassers.

14. Verfahren nach dem vorhergehenden Anspruch, wobei
- eine Porenwasserprobe in einem bestimmten, zum Beispiel regelmäßigen, Intervall durch das offene Ende (24, 124) einer der Röhren (21, 121) entnommen wird;
- die Probe analysiert wird, um die Bestimmung der Wasserqualität, zum Beispiel das Vorhandensein einer Schadstoffquelle, zu ermöglichen.

## Claims

1. A device (1, 10, 100) intended to be placed in the bed of a watercourse (2) or of a body of water to determine the reproductive success of fish, for example salmons, the device (1, 10, 100) comprising
a bottom (3, 13, 103) and a cover (4, 14, 104) positioned facing said bottom (3, 13, 103),
the bottom (3, 13, 103) and the cover (4, 14, 104) being reversibly linked by a lateral face (5, 15, 105) extending from the bottom (3, 13, 103) towards the cover (4, 14, 104) according to a longitudinal axis (L) of the device (1, 10, 100) so as to define a chamber (6, 16, 106),
said lateral face (5, 15, 105) comprising pores (7, 17, 127) intended to allow water of the watercourse (2) or of the body of water to pass into the chamber (6, 16, 116),
the device (1, 10, 100) further comprising several hollow tubes (21, 121) extending between the bottom (3, 13, 103) and the cover (4, 14, 104) along the longitudinal axis (L), each tube (21, 121) having a so-called closed end (22, 122) which is blocked by the bottom (3, 13, 103) and an opposite so-called open end (23, 123) passing through the cover (4, 14, 104) and emerging into the watercourse (2) or the body of water when the device (1, 10, 100) is immersed,
each tube (21, 121) comprising several openings (24, 124) arranged in the wall of the tube (21, 121) intended to allow water from the chamber (6, 16, 116) to pass into the tube (21, 121), so as to create a fluid connection between the open end of said tubes (21, 121) and the pores (7, 17, 127) of the lateral face (5, 15, 105) via said openings (24, 124).

2. The device (1, 10, 100) according to claim 1, wherein the openings (24, 124) are distributed over the length of the tubes (21, 121) to allow water to be taken at different depths, for example the device (1, 10, 100) comprises three tubes (1, 10, 100), a first tube (21a) where the openings (24) are close to the cover (14), a second tube (21c) where the openings (24) are close to the bottom (13) and a third tube (21b) where the openings (24) are halfway between the bottom (13) and the cover (14).

3. The device (1, 10, 100) according to any of the preceding claims, wherein each opening (24, 124) has a sufficiently small diameter to prevent the passage of eggs when there are fish eggs placed in the chamber (6, 16, 116) but sufficiently large to allow the passage of water, said openings having a diameter comprised between approximately 0.1 mm and 1.6 mm, preferably each opening has a diameter comprised between 0.50 mm and 1.5 mm, more preferably between 1.0 and 1.5 mm, for example approximately 1.0 or approximately 1.5 mm.

4. The device (1, 10, 100) according to any of the preceding claims, wherein the device (1, 10, 100) is cylindrical in shape, the bottom (3, 13, 113) and the cover (4, 14, 114) defining the bases of said cylinder, said bases being connected by the circular lateral face (5, 15, 115) according to the height of said cylinder making it possible to define a cylindrical chamber.

5. The device (1, 10, 100) according to any of the preceding claims, wherein the device (1, 10, 100) further comprises a reducer coupled around the periphery of the chamber (6, 16, 116) near the bottom (3, 13, 113) when the bottom (3, 13, 113) is coupled to the lateral face (5, 15, 115),
the reducer comprising an inclined plane (126) facing the cover (4, 14, 114) when the cover (4, 14, 114) is coupled to the lateral face (5, 15, 115),
said reducer making it possible to facilitate the flow of the content of the chamber (6, 16, 116) when the bottom (3, 13, 113) of the chamber (6, 16, 116) is open, for example the reducer is a ring (127).

6. The device (1, 10, 100) according to any of the preceding claims, wherein the device (1, 10, 100) comprises between 2 and 10 tubes (21, 121), preferably between 2 and 6 tubes (21, 121), preferably between 2 and 4 tubes (21, 121), for example 3 tubes (21, 121).

7. The device (1, 10, 100) according to any of the preceding claims, wherein each tube (21, 121) comprises between 1 and 20 openings (24, 124), preferably between 4 and 9 openings (24, 124), preferably between 5 and 7 openings (24, 124), for example 6 openings (24, 124).

8. The device (1, 10, 100) according to any of the preceding claims, wherein at least one of said tubes (21, 121) comprises two openings (24, 124) opposite to each other on either side of said tube so as to define a through channel perpendicular to the longitudinal axis of the tube (L).

9. The device (1, 10, 100) according to any of the preceding claims, wherein each opening (24, 124) has a diameter comprised between approximately 0.50 mm and 1.5 mm, preferably between 1.0 and 1.5 mm, for example approximately 1.0 mm or approximately 1.5 mm.

10. The device (1, 10, 100) according to any of the preceding claims, wherein the bottom (3, 13, 113) and/or the cover (4, 14, 114) comprises reversible coupling means for securing the bottom and/or the cover with the face, for example by screwing.

11. The device (1, 10, 100) according to any of the preceding claims, wherein the device (1, 10, 100) comprises a capsule, for example a dome (9, 109), coupled to the cover (4, 114) and defining an external chamber (8, 108) between the capsule and said cover (4, 114), the cover (4, 114) comprising a central channel (126) to ensure a fluid connection between the chamber (116) and the external chamber (108).

12. A method for taking a sample of interstitial water from a watercourse or a body of water, the method comprising:
- Providing a device (10) according to any of claims 1 to 10;
- Opening the cover (14) and introducing into the chamber (16) several constituents taken from the bed of the watercourse or of the body of water, for example gravel;
- Closing the cover (14):
- Placing the device (10) in the bed of the watercourse or of the body of water so that the cover (14) is flush with the surface of the bed of the watercourse or of the body of water, the chamber (16) being recessed into the bed so that the interstitial water penetrates the chamber (16) through the pores (17) of the lateral face (15);
- Taking a sample of interstitial water through the open end (23) of one of the tubes (21).

13. A method for determining the quality of water of watercourses or bodies of water, preferably using biological indicators, the method comprising:
- Providing a device (1, 100) according to any of claims 1 to 10;
- Opening the cover (4, 114);
- Introducing into the chamber (16, 116) several constituents taken from the bed of the watercourse or of the body of water, for example gravel, and several fish eggs, for example salmons,
- Closing the cover (14, 114);
- Placing the device (14, 114) in the bed of the watercourse or of the body of water so that the cover (14, 114) is flush with the surface of the bed of the watercourse or of the body of water, the chamber (16, 116) being recessed into the bed so that interstitial water penetrates the chamber (16, 116) through the pores (17, 117) of the lateral face (15, 115);
- Monitoring the development of eggs of the chamber (16, 116) and based on this development, determining the quality of the water of the watercourse or of the body of water, in particular of the interstitial water;

14. The method according to the preceding claim, wherein,
- a sample of interstitial water is taken at a determined, for example regular, interval through the open end (24, 124) of one of the tubes (21, 121);
- the sample is analyzed to enable the determination of water quality, for example the presence of a source of pollution.
